# EUROPEAN PATENT APPLICATION

(11) **EP 1 688 135 A1**
(43) Date of publication of application: **09.08.2006**
(21) Application number: 04723222.8
(22) Date of filing: 25.03.2004
(51) Int. Cl.: A61K 31/047, A61P 9/14

(54) **SCLEROSING SOLUTION**

(30) Priority: 08.04.2003 ES 200300824
(71) Applicant: Cabrera Garrido, Antonio Luis, 18005 Granada (ES)
(72) Inventor: Cabrera Garrido, Antonio Luis, 18005 Granada (ES)
(86) International application number: PCT/ES2004/000133
(87) International publication number: WO 2004/089358

(57) **Abstract**

The present invention relates to a new extremely effective and high safety sclerosing solution obtained by mixing:
sodium tetradecyl sulphate (STS), polidocanol (POL), glycerol (Gly)and/or hydroxyethyl starch (HES) in bidistilled water. The concentration of each compound expressed as wtlwt % of the total formulation is ranged between : 1 to 10 for STS, 1 to 10 for POL, 10 to 50 for Gly and 6 to 20 for HES. The solution is prepared with the use of sterile equipment in an aseptic environment.

## Description

### OBJECT OF THE INVENTION

This descriptive report refers to an application for an Invention Patent, corresponding to a sclerosing solution. Its obvious purpose lies in its application as an effect and safe sclerosing solution to treat varicose veins, venous angiomas, malformations and oesophageal varices.

### FIELD OF THE INVENTION

This invention is applicable in the industry that deals with the manufacture of pharmaceutical and medicinal products.

### BACKGROUND TO THE INVENTION

In many countries varicose veins are probably the most common disorder that presents in general medicine, and account for a mean of approximately 30% of the total time spent on caring for patients with venous ulcers.

However, on the other hand, relatively little investigation has been reported on a disease of such magnitude.

It has been demonstrated that scierotherapy is the most widely-used technique for varicose vein treatment, and sclerosing solutions are used directly inside the veins.

The mechanism of action of sclerosing solutions is based on causing endothelial damage, known as endosclerosis, by a group of detergent sclerosants that act on the venous endothelium, dissolving the lipoproteins from the endothelial surface, and causing venous spasm followed by thrombosis of the blood that comes into contact with this unprotected endothelial surface. The level of damage to the blood vessel wall determines the effectiveness of the solution.

Numerous sclerosing agents have been tested in sclerotherapy, and the ones used most commonly today are Polidocanol in Europe and Sodium Tetradecyl Sulfate in the United States. Both are known as detergent sclerosants because they are amphyphilic, inactive substances when they are in solution, but are biologically active when they form micelles.

However, neither Polidocanol nor Sodium Tetradecyl Sulfate have been accepted or emerged as ideal agents. Glycerine can also be considered as a detergent. It is specifically used in small vessels since it has less activity than the aforementioned products.

The administration of detergents in the form of foam dispersion has advantages over the liquid form, such as: better tolerance to extravasation, larger volume for the same quantity of active substance, ultrasound control of its application since foam is visible on ultrasound, etc. It should be indicated that detergents can generally be made into foam by simple agitation.

In a recent study conducted on 129 patients, it was found that Sodium Tetradecyl Sulfate (STS) and Polidocanol (POL) were equally safe, and, similarly, there are numerous epidemiological studies that conclude that glycerine (Gly) and/or hydroxyethyl starch (HES) are safe and easy to use.

The obvious solution to today's problems should lie in the application of an effective and safe sclerosing solution, based on the fact that the combination of POL and STS have a synergetic effect, attaining greater efficacy in comparison with POL or STS when applied separately; and furthermore, Gly and HES are added to the mixture because of their capacity to modify the viscosity of the solution, increasing the time that the sclerosant is in contact with the endothelium, thus increasing the surfactant action of the two detergents, and also Gly and HES increase the foam's stability and its consistency.

Furthermore, the applicant should also indicate that in varicose vein sclerotherapy, the fibrous healing response is subsequent to a physical-chemical aggression of the active substance on the venous wall.

The endothelium is the target of pharmacological action; it is the protective element that has to be destroyed in order to eliminate the venous path. Associated thrombosis is a constant parameter of greater or lesser magnitude, together with the endothelial inflammatory reaction and the environment in which thrombosis occurs.

Inflammation and thrombosis are not desirable effects that are sought, but they always appear to a greater or lesser extent in all sclerotherapy.

Elastic compression reduces thrombosis volume and significantly moderates the inflammatory response. Throughout this process the venous wall is the number one therapeutic objective and is the leading defendant in the fight against the aggression and in reacting against it to a greater or lesser extent.

Venous tone provides significant assistance that can be seen in the clear advantages when therapy acts at cold times of year.

The transitory venous spasm that occurs after application of the sclerosant is an indicator of the irritant power of the pharmacological action and the way in which the venous wall defends itself against this action. On a medium term it could be considered as assistance towards bringing the venous walls together, reducing the lumen and thrombotic contents with its parallel inflammatory response.

As the venous wall spasm continues in the course of time, it has a similar utility as the elastic compression, and in this respect, the potent effect of sclerosis can be potenciated with venotonics (such as dihydroergotamin).

Topical antiinflammatories (dexamethasone) with their persistent effect, as well as fibrinolysis inhibitors (tranexamic acid), have a potent action by correcting physiopathological parameters that are undesirable when the goal is sclerosis.

However, the applicant does not know of any invention that exists at present that presents these ideal characteristics.

### DESCRIPTION OF THE INVENTION

The sclerosing solution proposed in this invention consists of a solution that is principally for application in varicose veins and other similar disorders. It has been found to be effective and very safe.

More specifically, the sclerosing solution that forms the object of this invention is formed by mixing sodium tetradecyl sulfate (STS) and polidocanol (POL), glycerine (Gly) and/or hydroxyethyl starch (HES) in bidistilled water.

### PREFERRED EMBODIMENT OF THE INVENTION

The advocated sclerosing solution is made up of the following components:
- Sodium tetradecyl sulfate (STS), 1 to 10% in order to obtain any given quantity.
- Polidocanol (POL), 1 to 10% in order to obtain any given quantity.
- Glycerine (Gly), 10 to 50% in order to obtain any given quantity.
- Hydroxyethyl starch (HES), 6 to 20% in order to obtain any given quantity.

It should be indicated that all these elements shall be added to distilled water, and the solution shall be prepared using sterile materials in an aseptic atmosphere.

It should be indicated that if a specialist considers it necessary, in view of the thickness of a certain vein, a potentiator based on dihydroergotamin may be added to the sclerosing solution, since it will make the sclerosing formula ten times more efficient. It should be made quite clear and unquestionable that the potentiator based on dihydroergotamin does not form part of the sclerosing solution, but is a product that potentiates the latter's action in certain cases.

## Claims

1. A sclerosing solution principally designed for the treatment of varicose veins, venous angiomas, malformations and oesophageal varices, **characterised in that** it is formed by mixing sodium tetradecyl sulfate (STS) and polidocanol (POL), glycerine (Gly) and/or hydroxyethyl starch (HES) in bidistilled water.

2. The sclerosing solution, according to claim one, is **characterised in that** the sodium tetradecyl sulfate (STS) is added to the distilled water in order to obtain any given quantity from 1 to 10%.

3. The sclerosing solution, according to claim one, is **characterised in that** the polidocanol (POL) is added to the distilled water in order to obtain any given quantity from 1 to 10%.

4. The sclerosing solution, according to claim one, is **characterised in that** the glycerine (Gly) is added to the distilled water in order to obtain any given quantity from 10 to 50%.
